# FASCICULE DE BREVET EUROPEEN

(11) **EP 3 801 580 B1**
(45) Date de publication et mention de la délivrance du brevet: **30.08.2023**
(21) Numéro de dépôt: 19734252.0
(22) Date de dépôt: 31.05.2019
(51) Int. Cl.: A61K 35/74, A61P 17/08, A61P 17/06, A61P 17/04, A61P 37/06, A61K 31/7105, A61K 9/00, C12N 1/20

(54) **EXTRAIT ET COMPOSITION DERMATOLOGIQUE LE COMPRENANT POUR LE TRAITEMENT DES PEAUX SENSIBLES**
EXTRAKT UND DERMATOLOGISCHE ZUSAMMENSETZUNG DAMIT ZUR BEHANDLUNG EMPFINDLICHER HAUT
EXTRACT AND DERMATOLOGICAL COMPOSITION COMPRISING SAME, FOR TREATING SENSITIVE SKIN

(30) Priorité: 01.06.2018 FR 1854794
(43) Date de publication de la demande: 14.04.2021
(73) Titulaire: Pierre Fabre Dermo-Cosmétique, 92100 Boulogne-Billancourt (FR)
(72) Inventeur: CASTEX-RIZZI, Nathalie, 31770 COLOMIERS (FR); CHOL, Bertrand, 74350 VILLY LE PELLOUX (FR); LESTIENNE, Fabrice, 31810 VERNET (FR)
(74) Mandataire: Regimbeau
(86) Numéro de dépôt international: PCT/EP2019/064211
(87) Numéro de publication internationale: WO 2019/229248

(56) Documents cités:
- EP-A1- 2 018 891
- WO-A1-2012/085182
- US-A1- 2018 125 951
- Ying Chen ET AL: "Brain-Skin Connection: Stress, Inflammation and Skin Aging", Inflammation & Allergy -Drug Targets, 1 janvier 2014 (2014-01-01), pages 177-190, XP055548070, Extrait de l'Internet: URL:http://www.eurekaselect.com/122325/art icle [extrait le 2019-01-28]

## Description

### DOMAINE DE L'INVENTION

La présente description concerne un nouvel extrait bactérien qui peut être utile dans la protection et/ou le traitement de la peau sensible et/ou de la peau intolérante réactive, notamment par une action ciblée sur l'inflammation neurogène cutanée. La description a également pour objet des compositions cosmétiques ou dermatologiques comprenant un tel extrait bactérien comme agent actif.

### ETAT DE LA TECHNIQUE ANTERIEURE

L'épiderme est un épithélium pluristratifié qui exerce une fonction barrière de protection contre son environnement et ses agressions. Parmi ses multiples fonctions physiologiques, l'épiderme possède celle de constituer une barrière à la fois biologique, physique et chimique contre l'invasion de l'organisme par les microorganismes. Cette propriété de barrière physique de l'épiderme est notamment liée à sa structure. Ainsi l'épiderme est conventionnellement divisé en une couche basale de kératinocytes constituant la couche germinative de l'épiderme, une couche dite épineuse constituée de plusieurs couches de cellules polyédriques et enfin, un ensemble de couches supérieures appelé couche cornée (ou *stratum corneum*), constituée de kératinocytes au stade terminal de leur différenciation appelés cornéocytes. Les propriétés de barrière chimique de l'épiderme dépendent, notamment, de la libération à la surface de ce dernier de nombreux peptides antimicrobiens. Un dysfonctionnement de l'organisation structurelle cellulaire épidermique, ou un défaut de la fonction barrière chimique de l'épiderme, peut se traduire par un état inflammatoire cutané.

L'inflammation est une réaction de défense immunitaire normale de l'organisme à une agression de type : infectieuse, thermique, mécanique, chimique, lésionnelle ou encore allergique. Elle se caractérise en quatre points qui sont : la rougeur, la chaleur, le gonflement et la douleur. L'inflammation cutanée aigüe est une réponse immédiate à un agent agresseur, de courte durée (quelques jours ou semaines), d'installation souvent brutale et caractérisée par un gonflement intense. Les inflammations aigües guérissent spontanément ou avec un traitement. La chronicité apparaît lorsque l'inflammation ne guérit pas spontanément, persiste ou s'aggrave pendant plusieurs mois voire plusieurs années. La réaction inflammatoire est un processus dynamique comportant plusieurs étapes successives : vasculaire (vasodilatation), la diapédèse des leucocytes et le chimiotactisme des cellules immunitaires et pour finir la détersion. La diapédèse leucocytaire correspond à la traversée active des parois vasculaires et à l'accumulation de cellules immunitaires circulantes, les lymphocytes, les neutrophiles et les monocytes dans le foyer lésionnel. Les neutrophiles ont pour fonction d'attirer d'autres cellules inflammatoires par chimiotactisme et de nettoyer le site lésé en sécrétant des substances antimicrobiennes et des protéases. Les monocytes migrent par chimiotactisme et se différencient en macrophages nettoyant la zone lésée. Ils sécrètent des facteurs de croissances, des cytokines inflammatoires, comme IL-1, notamment IL-Iβ, TNFα, des protéases, des prostaglandines et des IFNs permettant le maintien et/ou l'amplification de l'inflammation. La détersion est consécutive à la phase vasculaire et contemporaine de la diapédèse leucocytaire. Il s'agit de l'élimination des tissus nécrosés et des agents pathogènes.

L'inflammation neurogène cutanée se définit comme l'induction et/ou l'amplification d'un processus inflammatoire primaire par les terminaisons nerveuses, ainsi il s'agit d'une inflammation de la peau induite par l'activation des fibres nerveuses intra-épidermiques qui sécrètent des neuropeptides tels que la substance P, qui jouerait notamment un rôle de cytokine inflammatoire (Ying Chen et al., Inflammation & Allergy - Drug Targets, 1 janvier 2014, 177-190). L'inflammation neurogène cutanée est particulièrement impliquée dans les peaux sensibles, les peaux intolérantes réactives voire dans des dermatoses inflammatoires prurigineuses. Le prurit est défini comme une sensation déplaisante qui provoque le besoin de se gratter. Il émerge depuis peu la notion de pruricepteurs, récepteurs spécifiques permettant de percevoir le prurit, mais leur distinction de la famille des récepteurs nociceptifs reste encore débattue. Ces pruricepteurs utilisent les fibres intra-épidermiques de type Ao et surtout celles de type C. Ils sécrètent des neuropeptides : la substance P, le calcitonin gene related peptide (CGRP) et le vasoactive intestinal peptide (VIP). Depuis peu, le rôle des protéases (trypsine, cathepsine G, thrombine etc.) dans l'induction du prurit a été clairement établi. En effet, leur récepteur PAR-2 a été défini comme la deuxième grande voie d'activation du prurit (Misery et al., Nat. Rev. Neurol 10, 408-416, 2014).

Les fibres nerveuses intradermiques interagissent directement ou indirectement avec les cellules cutanées et les cellules du système endocrinien, lymphatique et immunitaire. Ces communications ont conduit à la définition d'un système neuro-immuno-endocrino-cutané. Pour fonctionner, ce système nécessite un langage commun constitué de molécules de différentes natures, les neuromédiateurs, les cytokines et des facteurs de croissance. Ces molécules sont synthétisées et libérées par les cellules de la peau, les cellules immunitaires résidentes et recrutées, ainsi que par les terminaisons nerveuses intra-épidermiques. Les cellules épidermiques et dermiques peuvent également produire des neuromédiateurs, des enzymes, des neurotrophines, des cytokines, des chémokines et des facteurs de croissance. Ces médiateurs régulent l'innervation cutanée et la réponse inflammatoire. En cas d'inflammation, les cellules immunitaires en transit ou présentes constitutivement dans la peau, peuvent s'activer sous l'action de ces médiateurs sécrétées par les terminaisons nerveuses sensorielles et les cellules de la peau. Ce processus conduit à une seconde vague de libération de cytokines et neuromédiateurs, qui engendrent une boucle d'amplification de l'inflammation.

Un nouveau concept émerge depuis peu, suggérant que les kératinocytes sont également des acteurs majeurs de l'inflammation neurogène cutanée.

L'inflammation neurogène cutanée générée par des neuropeptides est impliquée dans la réactivité des peaux sensibles et aussi des peaux intolérantes. La notion de peau sensible reflète le niveau de sensibilité de la peau de chacun. S'il est possible d'avoir une peau sensible à tout âge, cela est extrêmement répandu chez les bébés et les personnes âgées. La peau des bébés a une épaisseur représentant environ un cinquième de celle de la peau des adultes, elle est par conséquent extrêmement sensible aux agressions chimiques, physiques et microbiennes, ainsi qu'aux rayons UV. La fonction de barrière de la peau des adultes, quant à elle, s'affaiblit progressivement avec l'âge, parallèlement au ralentissement des processus métaboliques. Le vieillissement de la peau entraîne peu à peu une déficience en lipides, ce qui la rend plus facilement irritable par les substances alcalines telles que le savon.

Lorsque la peau présente un seuil de sensibilité très bas, c'est-à-dire qu'elle réagit de manière exacerbée à la moindre agression extérieure, on parlera de peau intolérante, voire de peau intolérante réactive. Les peaux intolérantes sont plus vulnérables aux agressions extérieures et se caractérisent par un inconfort quotidien et une forte irritabilité. Certains signes, plus ou moins marqués, permettent de les reconnaître. La peau intolérante du visage présente par exemple des rougeurs et des picotements. Elle tire, chauffe ou démange. La sensation de démangeaison peut être traitée par divers moyens notamment par des dérivés de la toxine de clostridium (US2018/125951). Elle peut également procurer des sensations de brûlure. Les peaux intolérantes présentent généralement un terrain allergique et sont par conséquent, particulièrement sensibles aux composants des soins cosmétiques.

La peau sensible est en fait une peau sujette aux picotements, échauffements, fourmillements et démangeaisons, parfois accompagnées de rougeurs. Ces sensations d'inconfort apparaissent de façon exacerbée en réaction à des stimuli qui ne déclencheraient pas d'irritation sur une peau normale. Cette hyper-sensibilité de la peau résulte d'une diminution de son seuil de tolérance. Plus la peau est sensible, plus son seuil de tolérance est faible et lorsque le seuil de tolérance est au plus bas, on parlera de peau intolérante. Cette hyper-sensibilité peut s'expliquer par différents facteurs :
- Une réaction inflammatoire qui se développe au contact de substances chimiques irritantes comme certains savons, les détergents ménagers ou la pollution ; le seuil déclenchant des ces substances permettant ainsi d'évoquer la peau sensible ou la peau intolérante.
- Une altération de la fonction barrière de l'épiderme. Ce phénomène favorise alors une déshydratation de la peau et surtout la pénétration d'agents potentiellement irritants ;
- Des facteurs psychologiques, comme le stress ;
- Des facteurs hormonaux ;
- Des facteurs physiques comme le soleil, les changements de température (chaud/froid), le vent, la climatisation, le chauffage, l'eau calcaire.

La peau est recouverte d'un film protecteur, appelé film hydrolipidique de surface. Ce film en constitue la barrière la plus externe, ainsi que la plus fragile, la plus perturbée et la plus représentative de la santé de la peau ; il est constitué en grande partie des corps gras excrétés par les glandes sébacées et des lipides provenant de la dégradation des cellules lors de la phase de kératinisation des cellules cornées, ainsi que de composés hydrophiles, tels que l'eau de la sueur, le glycérol, l'urée, les facteurs naturels d'hydratation de la peau, les sels, les métabolites de la flore cutanée, etc. Ce film de surface est très exposé et très sensible aux stress environnementaux, aux habitudes d'hygiène, et à l'état de la peau. Il s'avère important de préserver, et même d'améliorer, cette fonction barrière, et ce d'autant plus pour les peaux les plus sensibles. Et, s'il est connu que les populations à peaux intolérantes dont la barrière cutanée est fragilisée nécessitent des soins avec des agents hydrolipidiques physio-mimétiques, notamment des agents émollients et hydratants physiologiques, il est en outre important d'éviter la mise en contact de la peau avec toute substance susceptible de dégrader le film hydrolipidique de surface, telle qu'un agent tensioactif ou un conservateur.

La peau sensible ou intolérante relève d'un mécanisme non allergique et implique une réaction inflammatoire sans reconnaissance d'un allergène spécifique.

Les mécanismes physiopathologiques de l'hyper-réactivité cutanée ne sont pas clairement élucidés mais on tend à identifier deux types de facteurs, qui peuvent être concourants. D'un côté il y a l'altération de la barrière cutanée *via* la perte en eau et l'altération des lipides intercornéocytaires. La peau devient plus sensible aux irritants et stimuli externes et l'irritation, même minime, entraine le relargage de cytokines inflammatoires et de composés de la cascade arachidonique. D'un autre côté, un trouble neurologique peut être responsable de la sensibilité, de la réactivité de la peau. Les fibres nerveuses parvenant jusqu'aux cellules de l'épiderme produisent, sous l'influence de stimuli externes, des neuromédiateurs (comme la substance P) à l'origine d'une inflammation neurogène, c'est l'inflammation neurogène cutanée.

On a donc un besoin en composition capable de traiter, prévenir, protéger les peaux sensibles, les peaux intolérantes dont la composante inflammatoire est une inflammation neurogène.

La présente description a pour objet de répondre à ces besoins, c'est-à-dire proposer une composition qui protège et/ou améliore l'état de la peau sensible, voire de la peau intolérante, en diminuant ou inhibant l'inflammation neurogène cutanée.

Une utilisation d'extrait bactérien cultivé en eau thermale pour réduire les rougeurs de la peau est décrit dans la demande EP2018891. Pour la première fois, la demanderesse a mis en évidence les propriétés bénéfiques dans la régénération tissulaire et la cicatrisation des lésions cutanées d'un extrait bactérien issu d'une souche bactérienne (ou bactérie) LMB64 isolée à partir d'une nappe phréatique. Cette bactérie a été décrite par la demanderesse dans la demande de brevet WO2012/085182. Plus particulièrement, étaient décrits et exemplifiés les extraits dénommés S0, E0 et ES0, respectivement constitués du surnageant de culture séparé de la biomasse, de la biomasse de cellules lysées, et du surnageant après incubation de la culture à pH basique pendant plusieurs heures. Les fractions E0 et ES0 étaient testées et il était montré que ces extraits E0 et ES0 avaient la capacité d'induction des cytokines et de maturation de cellules de Langherans (pour E0) ainsi que d'activation des récepteurs TLR2/TLR4/TLR5, d'antagonisme de récepteurs PARs et d'induction des peptides antimicrobiens (pour ES0). Ces résultats permettaient d'envisager l'utilisation de tels extraits dans le traitement de maladies inflammatoires comme le prurit, le psoriasis, l'eczéma ou encore la dermatite atopique.

### RESUME DE L'INVENTION

Les inventeurs ont de façon surprenante mis en évidence l'efficacité d'un nouvel extrait bactérien particulier dans la prévention et/ou le traitement de l'inflammation neurogène cutanée.

En effet, les inventeurs ont démontré que cet extrait bactérien présente un effet inhibiteur sur la libération par les kératinocytes d'IL-1β et de TNF-α induites par la substance P.

### DESCRIPTION DETAILLEE

L'invention est définie dans les revendications et tous les autres aspects exposés ici le sont à titre indicatif.

Selon un premier aspect, la présente description a pour objet un extrait bactérien selon la description ainsi que son utilisation dans la prévention et/ou le traitement de l'inflammation neurogène cutanée.

De manière particulière, la prévention et/ou le traitement de l'inflammation neurogène cutanée comprend, ou consiste en, la protection et/ou le traitement de la peau sensible ou de la peau intolérante.

De manière particulière, la prévention et/ou le traitement de l'inflammation neurogène cutanée comprend, ou consiste en, la protection et/ou le traitement de la peau sensible.

De manière particulière, la prévention et/ou le traitement de l'inflammation neurogène cutanée comprend, ou consiste en, la protection et/ou le traitement de la peau intolérante.

La bactérie LMB64 a été caractérisée et définie comme appartenant à la classe des Beta-proteobacteria, sous famille des Neisseriaceae, et probablement d'un nouveau genre non encore défini. L'analyse de la séquence du gène codant pour l'ARN ribosomique (ARNr) 16S a permis de situer cette bactérie proche des genres Chromobacterium, Paludimonas, Lutelia et Glubenkania, avec lesquels elle partage 95% de similarité de séquence. Cette bactérie, non-pathogène, est une bactérie à Gram négatif qui a été isolée à partir de la nappe phréatique. Plus particulièrement, la bactérie LMB64 se présente sous la forme d'un bâtonnet d'une longueur aux alentours de 2,3µm ± 0,3µm et d'une largeur aux alentours de 1,0µm ± 0,1µm. Une particularité de cette bactérie est la présence d'un flagelle polaire.

Le gène codant pour l'ARNr 16S a été presque totalement séquencé (1487 pb, correspondant à la séquence SEQ ID No. 1). La bactérie LMB64 possède un plasmide circulaire de 10948 pb. Ce plasmide a été entièrement séquencé et la séquence est représentée en la séquence SEQ ID No. 2.

Selon un autre aspect, une bactérie dont est issu un extrait bactérien selon la description comprend au moins un plasmide comprenant la séquence SEQ ID No. 2, ou toute séquence présentant au moins 80% d'identité avec la séquence SEQ ID No. 2, de manière avantageuse au moins 85%, au moins 90%, au moins 95%, encore au moins 97% et plus préférentiellement encore au moins 98% d'identité avec la séquence SEQ ID No. 2.

Aussi, une bactérie dont est issu l'extrait bactérien selon la description est une bactérie non-pathogène à Gram négatif appartenant à la classe des Betaproteobacteria, sous-famille des Neisseriaceae, ladite bactérie comprenant un ARNr 16S comprenant la séquence SEQ ID No. 1, ou toute séquence présentant au moins 80%, encore au moins 90%, au moins 95%, au moins 97% d'identité avec la séquence SEQ ID No. 1 et ladite bactérie comprenant au moins un plasmide comprenant la séquence SEQ ID No. 2, ou toute séquence présentant au moins 80% d'identité avec la séquence SEQ ID No. 2, de manière avantageuse au moins 85%, au moins 90%, au moins 95%, encore au moins 97% et plus préférentiellement encore au moins 98% d'identité avec la séquence SEQ ID No. 2.

A titre d'exemple une telle bactérie est représentée par la souche LMB64 qui a été déposée au nom de la demanderesse à la Collection Nationale de Cultures de Microorganismes (CNCM), Institut Pasteur, Paris, le 8 avril 2010 sous la référence I-4290.

Ayant ces informations génotypiques, associées aux caractéristiques de croissance en milieu non sulfuré, de la nature non filamenteuse de cette bactérie, un homme du métier n'aurait pas difficulté à trouver/identifier une autre bactérie permettant d'obtenir un extrait bactérien selon la description. Une telle identification d'une autre bactérie certes légèrement différente génotypiquement mais rassemblant les critères phénotypiques de la description quant à l'extrait bactérien peut être réalisé après un travail de sélection qui n'est en aucun cas insurmontable et ce sur la base des informations contenues dans la présente demande ainsi que celles contenues dans la demande WO2012/085182 associées aux connaissances générales de l'homme du métier.

Par « pourcentage d'identité » entre deux séquences d'acides nucléiques au sens de la présente description, on entend désigner un pourcentage de nucléotides identiques entre les deux séquences à comparer, obtenu après le meilleur alignement (alignement optimal, ce pourcentage étant purement statistique et les différences entre les deux séquences étant réparties au hasard et sur toute leur longueur). Les comparaisons de séquences entre deux séquences d'acides nucléiques sont traditionnellement réalisées en comparant ces séquences après les avoir alignées de manière optimale, ladite comparaison pouvant être réalisée par segment ou par « fenêtre de comparaison ». L'alignement optimal des séquences pour la comparaison peut être réalisé, outre manuellement, au moyen de l'algorithme d'homologie locale de Smith et Waterman (1981) [Ad. App. Math. 2 :482], au moyen de l'algorithme d'homologie locale de Neddleman et Wunsch (1970) [J. Mol. Biol. 48 : 443], au moyen de la méthode de recherche de similarité de Peason et Lipman (1988) [Proc. Natl. Acad. Sci. USA 85 : 2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences nucléiques est déterminé en comparant ces deux séquences alignées de manière optimale dans laquelle la séquence d'acides nucléiques à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide est identique entre les deux séquences, en divisant ce nombre de positions identiques par le nombre total de positions dans la fenêtre de comparaisons et en multipliant le résultat obtenu par 100 pour obtenir le pourcentage d'identité entre ces deux séquences.

Par exemple, on pourra utiliser le programme BLAST, « BLAST 2 séquences » (Tatusova et al., « Blast 2 sequences - anew tool for comparing protein and nucléotide sequences », FEMS Microbiol Lett. 174 :247-250) disponible sur le site http://www.ncbi.nlm.nih.gov/gorf/b12.html, les paramètres utilisés sont ceux donnés par défaut (en particulier pour les paramètres « open gap penaltie » : 5, et « extension gap penaltie » : 2 ; la matrice choisie est par exemple la matrice « BLOSUM 62 » proposée par le programme). Le pourcentage d'identité entre les deux séquences à comparer est calculé directement par le programme. Il est également possible d'utiliser d'autres programmes comme les logiciels « ALIGN » ou « Megalign » (DNASTAR).

Une bactérie selon la description, en particulier la bactérie LMB64, comprend au moins un plasmide comprenant la séquence SEQ ID No. 2, ou toute séquence présentant au moins 80%, de préférence 85%, 90%, 95% et 98% d'identité avec ladite séquence SEQ ID No. 2. D'autres caractéristiques de ladite bactérie, en particulier la bactérie LMB64, seront détaillées plus loin dans les exemples.

De manière générale, le terme « extrait bactérien selon la description » est utilisé pour décrire l'ensemble comprenant les composés solubles présents dans le cytosol de la bactérie, obtenus après l'isolement des cellules bactériennes du milieu de fermentation, leur lyse, en particulier par congélation-décongélation, une re-suspension dans un solvant aqueux et la récupération de la fraction liquide comprenant les composants cytosoliques, les composés intracellulaires solubles des cellules, les composés/molécules membranaires solubles, les composés/molécules transmembranaires solubles, les composés/molécules périplasmiques solubles ainsi que les composés/molécules flagellaires solubles, en particulier les protéines.

Par composés/molécules membranaires solubles, composés/molécules transmembranaires solubles, composés/molécules périplasmiques solubles ainsi que composés/molécules flagellaires solubles, il faut comprendre protéines et autres composés solubles contenus dans le cytoplasme, voire dans l'espace périplasmique, dans l'espace membranaire ou transmembranaire ou dans le flagelle, et qui sont libérés par la lyse d'une bactérie selon la description. Ces protéines et composés sont obtenus par le procédé selon la description, c'est à dire la récupération de la phase liquide suivant une séparation liquide/solide menée sur la masse cellulaire de bactéries lysées, par exemple par congélation-décongélation, après isolement de cette masse cellulaire du milieu de fermentation. Ces protéines ou composés intracellulaires cytosoliques, membranaires, périplasmiques et/ou flagellaires solubles comprennent par exemple les ribosomes, les enzymes associées au métabolisme cellulaire, des lipopolysaccharides, des sucres, des lipoprotéines, membranaires et périplasmiques, libérés par la lyse et solubles dans l'eau ou dans un solvant aqueux.

Les bactéries se multiplient par fission binaire, c'est-à-dire que chaque bactérie grandit puis se divise en deux cellules filles séparées par un septum de division formé par la paroi cellulaire. Durant la division, l'ADN se duplique ainsi que les autres constituants. Divers systèmes enzymatiques de synthèse et de dégradation participent à la division cellulaire.

La croissance bactérienne est l'accroissement ordonné de tous les composants de la bactérie. Elle aboutit à l'augmentation du nombre de bactéries. Au cours de la croissance, il se produit, d'une part, un appauvrissement du milieu de culture en nutriments et, d'autre part, un enrichissement en biomolécules sécrétées et excrétées dans le milieu de culture par la bactérie et solubilisées dans ce milieu ainsi qu'en sous-produits du métabolisme.

Par l'expression « milieu de culture », il faut comprendre tout milieu comprenant au moins les nutriments nécessaires pour la croissance et la multiplication des bactéries. Les bactéries peuvent être cultivées en milieu liquide, solide ou semi-liquide. De préférence, le milieu de culture est un milieu liquide permettant la croissance et la récupération de la biomasse et permettant l'obtention d'un extrait bactérien selon la description.

Un milieu de culture adéquat contient des nutriments favorisants la croissance et la multiplication des bactéries. De manière générale, un milieu de culture convenable pourra comprendre de l'eau une source de carbone, une source d'azote et des sels.

On pourra évoquer le « milieu de fermentation » ou culture bactérienne qui correspond au milieu de culture contenant les bactéries en fin de croissance et développement.

En pratique, l'extrait bactérien selon la description, en particulier un extrait de la bactérie LMB64, peut être obtenu à partir d'une culture d'une bactérie selon la description dans un milieu de culture permettant la croissance, le développement et la multiplication de ladite bactérie et la récupération des cellules après leur séparation de la phase liquide, par exemple centrifugation, sous la forme d'un culot ou biomasse. Cette biomasse est soumise à un traitement permettant de perméabiliser et détériorer les membranes et parois cellulaires - par exemple par congélation-décongélation. L'obtention de l'extrait selon la description peut se faire en reprenant la biomasse traitée, en particulier décongelée, avec un tampon basique puis un effectuant une séparation solide/liquide du mélange, par exemple par centrifugation. On obtient ainsi une phase aqueuse représentant l'extrait selon la description.

La récupération de la biomasse à partir du milieu de fermentation peut se faire par tout moyen de séparation liquide/solide. Plus particulièrement il est donc possible, par les techniques connues par l'homme de l'art, d'isoler la biomasse cellulaire contenant majoritairement des cellules entières, débris cellulaires comprenant des protéines de surface et/ou des protéines localisées dans l'espace périplasmique de la bactérie de la fraction liquide contenant les solutés résiduels du milieu de culture et les biomolécules excrétées par la bactérie et solubilisées dans le milieu de fermentation.

A titre illustratif, la séparation liquide/solide peut être réalisée par une technique choisie parmi : la centrifugation, la sédimentation, la filtration, l'ultrafiltration, la décantation.

De manière préférée, la séparation liquide/solide est réalisée par centrifugation milieu de fermentation contenant la culture bactérienne afin de séparer, d'un côté la phase solide, c'est-à-dire le culot de biomasse, contenant cellules et débris cellulaires et d'un autre côté la phase liquide, c'est-à-dire le surnageant comprenant les molécules solubles excrétées lors de la fermentation et composés résiduels du milieu de culture non consommés par la bactérie.

La phase solide, ie la biomasse obtenue, en particulier le culot de centrifugation, est soumise à une étape entraînant la rupture des membranes des cellules, par exemple une congélation, suivie d'une décongélation.

La congélation peut être réalisée à toute température négative permettant la solidification de l'eau, la formation de cristaux intracellulaires et intermembranaires et donc une rupture, au moins partielle, des membranes.

En particulier, la congélation peut être menée à une température de -10°C, voire de -20°C, voire encore de -30°C, de - 40°C, de -50°C de -60°C, voire encore de -80°C environ. Préférentiellement, la congélation est faite à -20°C environ.

La durée et la vitesse de congélation ne sont pas critiques en soi. La durée de congélation pourra être fonction de la température et par exemple une durée d'une à plusieurs heures est convenable. La phase solide mise congelée pourra aussi bien être conservées plusieurs jours, semaines ou mois même si ce n'est pas nécessaire.

La vitesse de congélation, ou de décongélation, n'est pas non plus critique et on cherchera des conditions permettant l'altération des parois et membranes bactériennes.

Par décongélation en entend un retour à une température positive permettant une fusion des cristaux de glace.

Cette étape de perméabilisation et rupture des parois et membranes peut aussi être réalisée par des moyens chimiques, ultrasoniques ou mécaniques tels que des détergents, agents chaotropiques, billes de verres, par exemple.

Cette étape permet la libération des composés intracellulaires cytoplasmiques solubles et qui sont donc présents dans cette phase solide de cellules lysées ou endommagées.

A cette phase solide est ensuite ajoutée une phase liquide sous la forme d'un solvant aqueux pour effectuer une resuspension des cellules lysées ou endommagées et extraire les composés solubles cytoplasmiques solubles dans ledit solvant.

Le solvant aqueux est préférentiellement un tampon, en particulier un tampon basique. D'une manière préférée ce tampon basique est un tampon Tris ou un tampon arginine ou un tampon Tris-arginine. De préférence il s'agit d'un tampon arginine. La concentration en arginine pourra être comprise entre 0.1 et 1 M, particulièrement environ 0.3 à 0.5 M. La concentration en Tris pourra être comprise entre 1 à 100 mM, particulièrement 20 mM. Le pH du tampon basique pourra être compris entre 8 et 12, et de préférence entre 9 et 11.

Cette étape de resuspension permet d'extraire les composés solubles cytoplasmiques contenus dans la biomasse de cellules lysées ou endommagées.

Enfin, on procède à la séparation liquide/solide pour récupérer une phase aqueuse liquide, en particulier une phase aqueuse liquide tamponnée, contenant des composés intracellulaires cytoplasmiques solubles.

Cette phase liquide obtenue représente ainsi l'extrait selon la description.

Le ratio phase solide/phase liquide aqueuse pour l'étape de resuspension peut être compris entre 1 et 10% w/v.

L'emploi d'un tampon basique permet de perméabiliser encore plus les membranes externes et de favoriser la diffusion des molécules depuis l'espace périplasmique vers le milieu liquide. L'emploi d'un tampon basique permet en outre de stabiliser les composés, en particulier les protéines solubles et d'éviter leur agrégation sur une longue période de stockage ainsi que leur dégradation par l'action des protéases.

Une ou plusieurs étapes de filtration peuvent être réalisées pour clarifier l'extrait et aboutir à un extrait selon la description, extrait purifié.

La filtration pourra être réalisée par tout moyen adéquat permettant une clarification de la phase liquide, ou de la phase liquide tamponnée. Une telle clarification par filtration permet l'élimination des particules en suspension qui n'auraient pas été éliminées lors de la seconde étape de séparation liquide/solide et vise à obtenir un extrait bactérien selon la description purifié, limpide.

La filtration peut être réalisée par tout moyen de filtration, ultrafiltration ou diafiltration.

De manière avantageuse la filtration est effectuée par filtration sur filtre ou cartouche à filtre présentant un seuil de 0,4µm, préférentiellement 0,2µm. Dans ce cas, l'extrait bactérien est caractérisé en ce que les composés présents dans l'extrait bactérien ont une taille inférieure ou égale à 0,2µm.

A titre préféré, il peut être utilisé des filtres ou préfiltres non chargés électrostatiquement afin d'éviter toute absorption des biomolécules responsables de tout ou partie de l'activité de l'extrait.

Les différentes étapes seront décrites plus en détails dans les exemples.

Selon un premier aspect, le procédé selon la description consiste en un procédé de préparation d'un extrait bactérien selon la description, ledit procédé comprenant les étapes de :
a) Mise en culture d'une bactérie selon la description, en particulier LMB64, dans un milieu approprié pour obtenir une culture bactérienne ;
b) séparation liquide/solide de ladite culture et élimination de la phase liquide ;
c) lyse cellulaire de la phase solide,
d) resuspension de la phase solide lysée dans une phase liquide aqueuse, préférentiellement tamponnée,
e) séparation liquide/solide et récupération de la phase liquide,
f) Optionnellement filtration de la phase liquide.
Selon un aspect préféré, l'étape c) de lyse cellulaire de la phase solide est réalisée par congélation suivie de décongélation.

L'invention a pour objet un extrait bactérien d'une bactérie représentée par la souche LMB64 qui a été déposée à la Collection Nationale de Cultures de Microorganismes (CNCM), Institut Pasteur, Paris, le 8 avril 2010 sous la référence I-4290, ledit extrait bactérien étant obtenu par un procédé comprenant les étapes de :
a. Mise en culture de ladite bactérie dans un milieu approprié pour obtenir une culture bactérienne ;
b. séparation liquide/solide de ladite culture et élimination de la phase liquide ;
c. lyse cellulaire de la phase solide,
d. resuspension de la phase solide lysée dans une phase liquide aqueuse, préférentiellement tamponnée,
e. séparation liquide/solide et récupération de la phase liquide,
f. Optionnellement filtration de la phase liquide, caractérisé en ce que la phase liquide aqueuse de l'étape de resuspension d) est un tampon basique choisi parmi tampon Tris, tampon arginine ou tampon Tris-arginine.

Selon un aspect particulier, la bactérie est une bactérie non pathogène à Gram négatif appartenant à la classe des betaproteobacteria, sous-famille des Neisseriaceae, comprenant un ARNr 16S comprenant la séquence SEQ ID No. 1, ou toute séquence présentant au moins 80% d'identité avec la séquence SEQ ID No. 1, plus particulièrement il s'agit de la bactérie LMB64.

De manière préférentielle, la bactérie comprend au moins un plasmide comprenant la séquence SEQ ID No.2, ou toute séquence présentant au moins 80% d'identité avec la séquence SEQ ID No.2.

Selon un mode de réalisation, l'extrait bactérien de l'invention est caractérisé en ce que la bactérie comprend au moins un plasmide comprenant la séquence SEQ ID No.2, ou toute séquence présentant au moins 80% d'identité avec la séquence SEQ ID No.2.

Selon un autre aspect, la présente description décrit un extrait bactérien obtenu, ou susceptible d'être obtenu, par un procédé selon la description tel que décrit supra.
Selon encore un autre aspect, la description décrit un extrait bactérien selon la description, en particulier un extrait obtenu ou susceptible d'être obtenu par un procédé selon la description, pour son utilisation pour la prévention, le traitement, la prévention et le traitement de l'inflammation neurogène cutanée.

Selon un mode de réalisation, l'extrait bactérien de l'invention est utilisé pour la prévention et/ou le traitement de l'inflammation neurogène cutanée. De manière préférée, l'extrait bactérien de l'invention est utilisé pour la prévention et le traitement de l'inflammation neurogène cutanée.

De manière avantageuse, l'inflammation neurogène cutanée comprend la peau sensible et/ou la peau intolérante.

Selon un autre mode de réalisation, l'extrait bactérien de l'invention est caractérisé en ce que l'inflammation neurogène cutanée comprend la peau sensible ou la peau intolérante.

Selon un autre aspect, la présente description décrit une composition cosmétique ou dermatologique comprenant au moins un extrait bactérien selon la description, avec au moins un excipient cosmétiquement ou dermatologiquement acceptable, pour son utilisation dans la prévention, le traitement, la prévention et le traitement de l'inflammation neurogène cutanée.

L'invention a en outre pour objet une composition cosmétique ou dermatologique comprenant un extrait bactérien selon l'invention et au moins un excipient cosmétiquement ou dermatologiquement acceptable.

Selon un mode de réalisation, la composition cosmétique ou dermatologique selon l'invention est caractérisée en ce qu'elle est appropriée à une application topique.

Selon un autre mode de réalisation, la composition cosmétique ou dermatologique selon l'invention est utilisée pour la prévention, le traitement ou la prévention et le traitement de l'inflammation neurogène cutanée.

Selon un autre aspect, la présente description décrit une composition cosmétique ou dermatologique comprenant au moins un extrait bactérien selon la description, avec au moins un excipient cosmétiquement ou dermatologiquement acceptable, pour son utilisation dans la protection et/ou le traitement des peaux sensibles ou intolérantes. En particulier il s'agit de peaux sensibles ou intolérantes ayant pour origine une inflammation neurogène cutanée.

Selon encore un autre mode de réalisation, la composition selon l'invention est caractérisée en ce que l'inflammation neurogène cutanée comprend la peau sensible ou la peau intolérante.

La présente description décrit également l'utilisation d'une composition cosmétique ou dermatologique comprenant au moins un extrait bactérien selon la description, avec au moins un excipient cosmétiquement ou dermatologiquement acceptable, pour la fabrication d'un médicament destiné à la prévention, le traitement, la prévention et le traitement de l'inflammation neurogène cutanée.

La description décrit également une méthode de prévention et/ou de traitement de l'inflammation neurogène cutanée comprenant l'administration à un individu en ayant besoin, d'une quantité efficace d'une composition cosmétique ou dermatologique comprenant au moins un extrait bactérien selon la description, avec au moins un excipient cosmétiquement ou dermatologiquement acceptable.

De manière avantageuse, l'inflammation neurogène cutanée comprend la peau sensible et/ou la peau intolérante.

Dans la présente description, on entend désigner par « cosmétiquement ou dermatologiquement acceptable » ce qui est utile dans la préparation d'une composition cosmétique ou dermatologique qui est généralement sûr, non toxique et ni biologiquement ni autrement non souhaitable et qui est acceptable pour une utilisation cosmétique ou dermatologique, notamment par application topique.

Selon un aspect particulier, la composition selon la description se présente sous une forme adaptée à une application topique.

Les compositions cosmétiques ou dermatologiques selon la description pourront se présenter sous les formes qui sont habituellement connues pour une administration topique, c'est-à-dire notamment les lotions, les mousses, les gels, les dispersions, les émulsions, les sprays, les sérums, les masques ou les crèmes, les gelées, notamment les gelées micellaires, avec des excipients permettant notamment une pénétration cutanée afin d'améliorer les propriétés et l'accessibilité du principe actif. Avantageusement, il s'agira d'une crème, une crème riche, une lotion, un soin yeux, un soin UV.

Ces compositions contiennent généralement, outre les composés de l'extrait bactérien selon la description, un milieu physiologiquement acceptable, en général à base d'eau ou de solvant, par exemple des alcools, des éthers ou des glycols. Elles peuvent également contenir des agents tensioactifs, des agents complexants, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Avantageusement, les compositions selon la présente description comprendront 0,05 à 10% en poids, de préférence 0,1 à 5% en poids, de manière encore préférée 0,5 à 3% en poids de l'extrait bactérien selon la description par rapport au poids total de la composition.

La composition selon la description apporte une protection qui reste confortable tout au long de la journée. Elle peut en particulier être appliquée aux peaux sensibles, les peaux réactives, et notamment aux peaux de bébé.

De préférence, la composition selon la présente description est utilisée pour prévenir, protéger et/ou traiter les peaux sensibles.

D'une manière générale, les peaux sensibles se définissent par une réactivité particulière de la peau. Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons ultra-violets ou infrarouges. Il existe également des facteurs associés avec l'âge et le type de peau. Ainsi les peaux sensibles sont plus fréquentes parmi les peaux sèches ou grasses que parmi les peaux normales. Au sens de la présente description, les peaux sensibles couvrent les peaux irritables et les peaux intolérantes.

De telles compositions peuvent être fabriquées selon des procédés bien connus de l'homme du métier.

Les différents objets de la présente description seront mieux compris à la lecture des exemples ci-dessous qui les illustrent.

### Exemple 1 : Culture de la bactérie LMB64

A titre d'exemple, des milieux de culture préférés contiennent du chlorure d'ammonium, du sulfate de magnésium et de l'extrait de levure. A noter également, comme cela ressort entre autres de la demande WO2012/085182, d'autres milieux similaires pourront être utilisés et doivent donc être considérés comme faisant partie intégrante de la présente description. Toute adaptation de l'Homme de l'art doit également être considérée comme partie de la description.

Un exemple de procédé de culture est décrit ci-dessous. Il convient de rappeler ici que cet exemple n'a de valeur qu'illustrative.

La souche LMB64 est cultivée en trois étapes, à savoir un premier inoculum, une préculture (ou préfermentation) en mode batch et enfin une culture mais en mode fed-batch (ajout de glucose).

Inoculum : Un tube de la WCB LMB64 est utilisé pour ensemencer un Erlenmeyer contenant 1000mL de milieu stérile. L'erlenmeyer est ensuite placé dans le shaker incubateur sous agitation. Lorsque la densité cellulaire du bouillon est suffisante, la culture est arrêtée. Les cellules sont alors mises en refroidissement jusqu'à transfert dans le préfermenteur.

Préculture : le préfermenteur est ensuite rempli avec environ 16 L de milieu puis stérilisée à plein.

Deux flacons satellites sont connectés sur le préfermenteur après stérilisation de la cuve puis des blocs d'ajout :
- Un flacon contenant une solution stérile de glucose à 50%. Cette solution (glucose batch préculture) est transférée immédiatement dans le milieu de culture pour atteindre la concentration initiale de glucose de 20g/L.
- L'erlenmeyer contenant l'inoculum décrit plus haut à l'étape Inoculum est ensemencé dans le préfermenteur.

La préculture est lancée puis régulée automatiquement. A titre d'exemple, il peut être mentionné les paramètres suivants : température, vitesse d'agitation, pression, débit d'air ou encore PO₂.

La croissance des cellules est suivie par une mesure de la densité optique à 620nm. La préculture est arrêtée par refroidissement lorsqu'elle atteint une densité suffisante.
Culture : le fermenteur est ensuite rempli avec 127 L de milieu ajusté à pH 7,0 puis stérilisé à plein. Trois flacons satellites sont utilisés :
- Un flacon contenant une solution stérile de glucose. Cette solution est transférée immédiatement dans le milieu de culture pour atteindre la concentration initiale de glucose de 20g/L.
- Un flacon d'antimousse. Cet antimousse sera ajouté automatiquement pendant la culture pour contrôler le niveau de mousse dans la cuve.
- Un flacon de glucose fedbatch. Cette solution sera ajoutée en cours de culture pour supporter la croissance des cellules.

La culture est lancée puis régulée automatiquement. A titre d'exemple, il peut également être mentionné les paramètres suivants : température, pH, agitation, pression, débit d'air Poz.

Après épuisement du glucose initialement présent dans le milieu (remontée de Po2), l'ajout de la solution glucose fedbatch est déclenché et permet une croissance des cellules à haute densité. La fermentation est arrêtée après consommation totale du glucose. A ce stade, le mout de fermentation est refroidi automatiquement. Tout au long de la culture, la croissance des cellules est suivie par une mesure de la densité optique à 620nm. La quantité de biomasse sèche (g/L) obtenue en fin de culture est déterminée en utilisant une méthode pondérale.

### Exemple 2 : extraction de la fraction.

L'exemple ci-dessous est donné à titre illustratif.

L'extrait bactérien est obtenu de manière générale après centrifugation du résultat de l'étape de culture afin d'éliminer le surnageant et de garder la biomasse, c'est à dire les cellules, les protéines de surface, les protéines localisées dans l'espace périplasmique et des protéines intracellulaires de la bactérie (présence due à l'étape de congélation). Pour l'étape de centrifugation, la ligne de transfert du fermenteur à la centrifugeuse est stérilisée. Le mout de fermentation est ensuite séparé par centrifugation continue sur une centrifugeuse. La centrifugation est conduite à 150 L/h (± 30 L/h) avec une vitesse de rotation du bol de 10900 ± 1000 rpm. Les cellules sont récupérées dans une poche à usage unique. Le surnageant est éliminé à l'upérisation. Cette étape de centrifugation est suivie d'une étape de congélation à -20°C pendant du culot pendant au moins 1 heure.

Cent dix litres de tampon d'extraction Tris Arginine sont stérilisés dans le fermenteur. Les cellules auparavant décongelées à température ambiante sont transférées dans le fermenteur via la pompe péristaltique. Le temps de contact nécessaire est compris entre 1 et 7 heures. La concentration cible en Tris et arginine après ajout dans la poche à usage unique est aux alentours de 0,3M en L-arginine et 20mM en Tris.

Le mout de fermentation est ensuite séparé par centrifugation continue sur une centrifugeuse. La centrifugation est conduite à 100 L/h (± 30 L/h) avec une vitesse de rotation du bol de 10900 ± 1000 rpm. Les débourbages partiels sont initiés automatiquement en fonction de la turbidité de l'effluent avec une consigne à 20% de turbidité. Une série de débourbages totaux est déclenchée manuellement à la moitié du volume à séparer. Le surnageant est récupéré dans une poche à usage unique. Les cellules sont éliminées à l'upérisation.

Deux étapes de filtration sont réalisées en ligne, afin de clarifier le surnageant et aboutir à un extrait bactérien exempt de germes. Le pilotage de la filtration est réalisé grâce au système de filtration/répartition. La cartouche de filtration en profondeur à usage unique est placée dans son carter de filtration. Les manifolds de filtration sont équipés de leurs manomètres permettant la sécurisation de l'étape de filtration. Le module de pré filtration est rincé avec environ 92L ± 5L d'eau purifiée puis la poche de produit à filtrer est connectée et mise sous agitation. Enfin, la cartouche de filtration 0,2µm 30" en PES est connectée au reste du système de filtration. La filtration est réalisée à un débit initial de 240L/h ± 10L grâce à une pompe péristaltique. Lorsque la pression en amont des filtres atteint 1,2 bars le débit de filtration est diminué. La totalité du produit filtré est récupéré stérilement dans un container équipé d'une poche à usage unique de 400L. La poche de produit filtré est pesée sur le plateau balance puis stockée à +5°C jusqu'à répartition. Après utilisation, le filtre stérilisant est déconnecté puis contrôlé par un test d'intégrité.

### Exemple 3 : Effet de l'extrait bactérien sur l'inflammation neurogène cutanée

Le but de cette étude est d'évaluer les propriétés modulatrices de l'extrait bactérien dans l'inflammation cutanée. Pour ce faire une approche expérimentale *in vitro* basée sur la mesure de production et de libération de TNF-α (Tumor Necrosis Factor-alpha) et d'IL-Iβ (Interleukin-1 beta) par des kératinocytes épidermiques humains, activés par la substance P, est proposée comme modèle in vitro de l'inflammation neurogène cutanée.

### Protocole

L'étude est réalisée sur des kératinocytes épidermiques normaux humains, ces cellules sont obtenues à partir de prépuces de nouveau-nés. Ces cellules sont mises en culture dans un milieu sans sérum selon des procédures classiques en laboratoire. Le dosage des marqueurs de l'inflammation neurogène cutanée est réalisé sur ces kératinocytes cultivés dans un milieu en absence (condition contrôle) ou en présence des composés à tester (extrait bactérien et produit de référence), et exposés ou pas à la substance P. Les kératinocytes sont traités 3 heures avant le stress induit par la substance P et durant les 24 heures suivantes. Les niveaux de cytokines pro inflammatoires (TNF-α et IL-1β) sont quantifiés 24h après l'induction du stress inflammatoire par l'ajout de la substance P (0,5µM). Simultanément, le composé CP96345, un inhibiteur sélectif NK-1R est testé à 1uM comme produit de référence. Chaque condition expérimentale est conduite sur deux donneurs différents de kératinocytes.

Les productions de TNF-α et IL-1β sont mesurées dans les milieux d'incubation et quantifiées par ELISA (Enzyme-Linked Immunosorbent Assay). L'analyse statistique (*p<0,05 ; **p<0,01 ou ***p<0,001) est déterminée sur le pourcentage d'inhibition en utilisant un test non-paramétrique car les données ne passent pas le test de normalité, suivi par le test de comparaison multiple de Dunn comme post-test.

Les résultats sur la libération IL-1β (pg/mg de protéine totale) sont résumés dans le tableau 1 ci-dessous :

| Groupes | conc | IL-1β (pg/ml) | | % Inh | Stats |
|---|---|---|---|---|---|
| | | moyenne | e.s. | | |
| Cont SP(-) | | 122,2 | 11,8 | - | P<0,001 |
| Cont SP(+) | | 241,2 | 18,3 | | |
| NK-1R inh | 1µm | 132,5 | 12,1 | 91 | P<0,05 |
| Composé | 1,64µg/ml | 143,2 | 9,2 | 80 | NS |
| | 5, 45pg/ml | 127,7 | 8,4 | 93 | P<0,05 |
| | 16,36µg/ml | 125,7 | 10,2 | 96 | P<0,01 |

| | | | | | |
|---|---|---|---|---|---|
| Conc : concentrations, Inh : inhibition, Stats : statistique versus Cont (+) ; E.s. : Erreur standard à la moyenne ; Cont : contrôle (sans produit à tester) ; SP (-) : sans substance P ; SP (+) : en présence de substance P. | | | | | |

L'exposition des kératinocytes à la substance P induit une libération importante et statistiquement significative d'IL-1β. Le traitement par le CP96345 à 1 µM réduit fortement (91%, P<0,05) la production d'IL-1β. Ce résultat est très probant, l'utilisation de cet inhibiteur NK-1R a permis de valider ce test. Le traitement des kératinocytes avec le composé permet une inhibition concentration-dépendante de la libération d'IL-1β induite par la substance P. Si la première concentration testée (1,64µg/ml de protéines) n'atteint pas la significativité, elle réduit tout de même de 80% cette libération d'IL-1β. Aux concentrations de 5,45µg/ml et 16.36µg/ml (µl/ml de protéine), cette inhibition est statistiquement significative (93% et 96% d'inhibition, p<0,05 et p<0,01, respectivement).

Les résultats sur la libération de TNF-α (pg/mg de protéine totale) sont résumés dans le tableau 2 ci-dessous :

| Groupes | conc | TNF-α (pg/ml) | | % Inh | Stats |
|---|---|---|---|---|---|
| | | moyenne | e.s. | | |
| Cont SP(-) | | 4,1 | 0,7 | - | P<0,01 |
| Cont SP(+) | | 9, 9 | 0,8 | | |
| NK-1R inh | 1µm | 4,6 | 0,9 | 90 | P<0,001 |
| extrait | 1,64ug/ml | 6,1 | 0,5 | 63 | NS |
| | 5,45µg/ml | 5,9 | 0, 6 | 68 | NS |
| | 16,36µg/ml | 5,4 | 0, 6 | 77 | P<0,01 |

| | | | | | |
|---|---|---|---|---|---|
| Conc : concentrations, Inh : inhibition, Stats : statistique versus Cont (+) ; E.s. : Erreur standard à la moyenne ; Cont : contrôle (sans produit à tester) ; SP (-) : sans substance P ; SP (+) : en présence de substance P. | | | | | |

L'exposition des kératinocytes à la substance P induit une libération importante et statistiquement significative de TNF-α. Le traitement par le CP96345 à 1 µM réduit fortement (90%, P<0,05) la production de TNF-α. Ce résultat est également très probant, l'utilisation de cet inhibiteur NK-1R a permis de valider ce test. Le traitement des kératinocytes avec l'extrait bactérien permet une inhibition concentration-dépendante de la libération de TNF-α induite par la substance P. La plus faible concentration testée de l'extrait bactérien inhibe la libération de TNF-α de 60%. Même si cette inhibition n'atteint pas le seuil de significativité, l'effet concentration-réponse mis en évidence montre clairement que l'extrait bactérien est très actif sur ce test. A la concentration de 16.36µg/ml de protéine, cette inhibition est statistiquement significative (77% d'inhibition p<0,01).

Ainsi les inventeurs mettent en évidence qu'un extrait bactérien est capable d'inhiber significativement la production de cytokines produites via la substance P, cet extrait bactérien est donc efficace dans le traitement de l'inflammation neurogène cutanée.

### Exemple 4 : Effet de l'extrait bactérien sur le profil d'expression génique de l'immunité innée dans un modèle de kératinocytes épidermiques normaux humains

La fonction barrière de la peau inclut aussi une défense contre les micro-organismes. L'épithélium joue un rôle actif dans les défenses innées de l'hôte. Les systèmes antimicrobiens cutanées reposent entre autres sur la présence de certains lipides de surface et de certaines protéines constitutives. Ces protéines possèdent des activités antimicrobiennes. De plus, l'acidification de la surface épidermique joue un rôle important sans la défense antimicrobienne cutanée. La peau agit ainsi non seulement comme une barrière physique, mais aussi comme une barrière chimique. Il existe également une composante adaptative de l'immunité innée reposant sur la sécrétion inductible de peptides antimicrobiens. Ces derniers jouent un rôle important comme médiateurs de l'inflammation en ayant des effets sur les cellules épithéliales et inflammatoires, en influençant la prolifération cellulaire et la production de cytokines. Leur mode d'action consiste à rompre la membrane plasmique des microbes infectieux ou à pénétrer dans le microorganisme afin d'interférer avec le métabolisme intracellulaire. Les peptides antimicrobiens les plus étudiés dans la peau sont les β-défensines et les cathélicidines. Les β-défensines humaines constituent la classe majeure des peptides antimicrobiens retrouvés dans les épithéliums humains et quatre d'entre elles ont été identifiées dans la peau, HBD 1-4. Bien qu'elles appartiennent à la même famille, elles sont régulées par des voix différentes. La β-défensine 2 humaine (hBD2), un peptide de 4kDa se liant à l'héparine est l'un des principaux peptides antimicrobiens cutanés. L'expression des peptides hBD2 est inductible soit par la sécrétion de cytokines (IL-1α et IL-Iβ et TNF-α) traduisant un état inflammatoire, soit par contact avec une bactérie ou un champignon.

Le but de cette étude est d'évaluer les effets de l'extrait bactérien sur l'expression génique de kératinocytes épidermiques normaux humains par une technique de RT-PCR sur 2 gènes reliés à l'immunité innée.

L'étude est réalisée sur des kératinocytes épidermiques normaux humains issus de trois donneurs. Ces cellules sont utilisées à leur troisième passage. Ces cellules sont mises en culture dans un milieu standard supplémenté en EGF (Epidermal Growth factor), de PE (Pituitary extract) et de gentamycine, selon des procédures classiques en laboratoire.

L'extrait bactérien est testé à trois concentrations, 0,4 ; 2 et 10pg/ml (pg/ml de protéines).

Simultanément, le chlorure de calcium est testé à 1,5mM comme produit de référence.

Les kératinocytes sont ensemencés en plaques de 24 puits (50000 cellules par puits) et mises en culture pendant 24 heures dans un milieu de culture. Le milieu est ensuite remplacé par un milieu d'essai contenant ou pas (condition contrôle, DMSO) l'extrait bactérien, ou le chlorure de calcium (produit de référence), les cellules sont incubées dans ces conditions pendant 48 heures. A la fin de cette période d'incubation, les cellules sont lavées dans une solution tamponnée et immédiatement congelées à une température de -80°C.

L'expression des marqueurs est analysée par la méthode de RT-qPCR sur l'ARN total extrait des cellules et ce dans chaque condition. L'ARN total est extrait dans chaque échantillon en utilisant le réactif Tripure Isolation^{®} selon les instructions du fournisseur. La quantité et la qualité d'ARN sont évaluées par électrophorèse capillaire. L'ADNc est synthétisé par transcription réverse de l'ARN total en présence d'oligo(dT) et de « Transcriptor Reverse Transcriptase ». La quantité d'ADNc est ensuite ajustée avant l'étape de PCR (Polymerase Chain Reaction). La PCR est réalisée en utilisant le système LightCycler^{®} de Roche selon les données du fournisseur.

Les données sont analysées par le logiciel Microsoft Excel.

L'incorporation de fluorescence dans l'ADN amplifié est continuellement mesurée pendant les cycles de PCR. Il en résulte dans une représentation graphique entre l'intensité de fluorescence et le nombre de cycles de PCR une valeur d'expression relative pour chaque marqueur.

La technique PCR utilisée dans cette étude inclut 2 gènes de référence (RPL13A et TBP), ces gènes sont utilisés pour la normalisation des données, puisque leur expression est constitutive et donc théoriquement stable. Par conséquent le niveau d'expression des gènes cibles est comparé à la moyenne du niveau d'expression de ces 2 marqueurs de référence pour toutes les conditions.

Le paramètre RQ (relative Quantification) est défini comme l'expression relative /100. Tous les résultats sont exprimés en facteur multiplicatif il représente le nombre de fois que le gène est surexprimé si RQ >1 ou sous-exprimé si RQ<1.

Le tableau 3 ci-dessous permet de classer les effets des conditions traitées vs contrôle

| Classification des effets | Facteur de multiplication (FC) |
|---|---|
| Forte stimulation | FC > 3 |
| Stimulation | 3 > FC > 2 |
| Légère stimulation, à confirmer | 2 > FC > 1,5 |
| - | 1,5 > FC > -1,5 |
| Légère inhibition, à confirmer | -1,5 > FC > -2 |
| Inhibition | -2 > FC > -3 |
| Forte inhibition | -3 > FC |
| Pas d'expression | Nombre de cycles > 33 |

L'analyse statistique est réalisée par une comparaison intergroupe par un test de Student non-apparié.

### Résultats

Le traitement des kératinocytes épidermiques normaux humains avec 1.5mM de chlorure de calcium utilisé comme contrôle positif induit une forte expression des gènes ciblés dans l'immunité innée, ce résultat permet de valider les conditions expérimentales (tableau 4).

| | | Chlorure de calcium (1,5 mM) | | |
|---|---|---|---|---|
| | gènes | Moyenne | Ecart type | sem |
| Immunité innée PAM | HBD2 | 5,8 | 6,1 | 3,5 |
| | S100A7 | 4,1 | 3,8 | 2,2 |

| | | | | |
|---|---|---|---|---|
| Pam : peptides antimicrobiens | | | | |

Le tableau 5 résume les effets de l'extrait bactérien aux trois concentrations testées

| | | Extrait bactérien (0,2µg/ml) | | |
|---|---|---|---|---|
| | gènes | Moyenne | Ecart type | sem |
| Immunité innée PAM | HBD2 | 4,5 | 3,16 | 1, 83 |
| | S100A7 | 2,1 | 0,39 | 0,23 |
| | | Extrait bactérien (1,0µg/ml) | | |
| Immunité innée PAM | HBD2 | 21, 0 | 12,29 | 7,10 |
| | S100A7 | 3,9 | 1, 61 | 0, 93 |
| | | Extrait bactérien (5,0µg/ml) | | |
| Immunité innée PAM | HBD2 | 596,4 | 546,48 | 315,51 |
| | S100A7 | 10,6 | 6,56 | 2,05 |

| | | | | |
|---|---|---|---|---|
| Pam : peptides antimicrobiens | | | | |

Les inventeurs mettent en évidence que toutes les concentrations testées de l'extrait bactérien induisent l'expression des gènes HBD2 et S100A7 et de façon-concentration-dépendante.

L'extrait bactérien a la capacité d'augmenter l'induction de peptides antimicrobiens. Par cette stimulation de l'immunité innée, les peptides antimicrobiens induisent la défense cutanée et participent à la protection de la barrière cutanée.

L'ensemble de ces résultats, c'est-à-dire une diminution de l'inflammation neurogène cutanée combinée à une aide à la protection de la barrière cutanée permet de montrer que l'extrait bactérien agit réellement sur les peaux sensibles et/ou intolérantes.

## Revendications

1. Extrait bactérien d'une bactérie représentée par la souche LMB64 qui a été déposée à la Collection Nationale de Cultures de Microorganismes (CNCM), Institut Pasteur, Paris, le 8 avril 2010 sous la référence I-4290, ledit extrait bactérien étant obtenu par un procédé comprenant les étapes de :
a. Mise en culture de ladite bactérie dans un milieu approprié pour obtenir une culture bactérienne ;
b. séparation liquide/solide de ladite culture et élimination de la phase liquide ;
c. lyse cellulaire de la phase solide,
d. resuspension de la phase solide lysée dans une phase liquide aqueuse, préférentiellement tamponnée,
e. séparation liquide/solide et récupération de la phase liquide,
f. Optionnellement filtration de la phase liquide,
**caractérisé en ce que** la phase liquide aqueuse de l'étape de resuspension d) est un tampon basique choisi parmi tampon Tris, tampon arginine ou tampon Tris-arginine.

2. Extrait bactérien selon la revendication 1, **caractérisé en ce que** la bactérie comprend au moins un plasmide comprenant la séquence SEQ ID No.2, ou toute séquence présentant au moins 80% d'identité avec la séquence SEQ ID No.2.

3. Extrait bactérien selon l'une des revendications 1 ou 2 pour son utilisation pour la prévention ou le traitement de l'inflammation neurogène cutanée.

4. Extrait pour son utilisation selon la revendication 3, **caractérisé en ce que** l'inflammation neurogène cutanée comprend la peau sensible ou la peau intolérante.

5. Composition cosmétique ou dermatologique comprenant un extrait bactérien selon l'une des revendications 1 ou 2 et au moins un excipient cosmétiquement ou dermatologiquement acceptable.

6. Composition cosmétique ou dermatologique selon la revendication 5, **caractérisée en ce qu'**elle est appropriée à une application topique.

7. Composition dermatologique selon la revendication 5 ou 6 pour son utilisation pour la prévention et le traitement de l'inflammation neurogène cutanée.

8. Composition pour son utilisation selon la revendication 7 **caractérisée en ce que** l'inflammation neurogène cutanée comprend la peau sensible ou la peau intolérante.

## Patentansprüche

1. Bakterieller Extrakt einer vom Stamm LMB64 vertretenen Bakterie, der bei der Collection Nationale de Culture de Microorganismes (CNCM), Institut Pasteur, Paris, am 8. April 2010 unter der Referenz I-4290 hinterlegt wurde, wobei der bakterielle Extrakt durch ein Verfahren erhalten wurde, das die folgenden Schritte umfasst:
a. Kultivieren der Bakterie in einem geeigneten Medium, um eine Bakterienkultur zu erhalten;
b. Fest-Flüssig-Trennung der Kultur und Entfernen der flüssigen Phase;
c. Zelllyse der festen Phase,
d. Resuspension der lysierten festen Phase in einer wässrigen flüssigen, vorzugsweise gepufferten Phase,
e. Fest-Flüssig-Trennung und Rückgewinnung der flüssigen Phase,
f. optional Filtration der flüssigen Phase,
**dadurch gekennzeichnet, dass** die wässrige flüssige Phase des Resuspensionsschritts d) ein basischer Puffer, ausgewählt aus Tris-Puffer, Arginen-Puffer oder Tris-Arginin-Puffer, ist.

2. Bakterieller Extrakt nach Anspruch 1, **dadurch gekennzeichnet, dass** die Bakterie mindestens ein Plasmid umfasst, das die Sequenz SEQ ID Nr. 2 oder jede Sequenz umfasst, die mindestens 80% Identität mit der Sequenz SEQ ID Nr. 2 aufweist.

3. Bakterieller Extrakt nach einem der Ansprüche 1 oder 2 für seine Verwendung zur Vorbeugung oder zur Behandlung der neurogenen Hautentzündung.

4. Extrakt für seine Verwendung nach Anspruch 3, **dadurch gekennzeichnet, dass** die neurogene Hautentzündung die empfindliche Haut oder die unverträgliche Haut umfasst.

5. Kosmetische oder dermatologische Zusammensetzung, umfassend einen bakteriellen Extrakt nach einem der Ansprüche 1 oder 2 und mindestens einen kosmetisch oder dermatologisch akzeptablen Hilfsstoff.

6. Kosmetische oder dermatologische Zusammensetzung nach Anspruch 5, **dadurch gekennzeichnet, dass** sie für eine topische Anwendung geeignet ist.

7. Dermatologische Zusammensetzung nach Anspruch 5 oder 6, für ihre Verwendung zur Vorbeugung oder zur Behandlung der neurogenen Hautentzündung.

8. Zusammensetzung für ihre Verwendung nach Anspruch 7, **dadurch gekennzeichnet, dass** die neurogene Hautentzündung die empfindliche Haut oder die unverträgliche Haut umfasst.

## Claims

1. Bacterial extract of a bacterium represented by strain LMB64 which was deposited at the Collection Nationale de Cultures de Microorganismes (CNCM), Institut Pasteur, Paris, on 8 April 2010 under number I-4290, said bacterial extract being obtained by a process comprising the steps of:
a. culture of said bacterium in a suitable medium to obtain a bacterial culture;
b. liquid/solid separation of said culture and removal of the liquid phase;
c. cell lysis of the solid phase,
d. resuspension of the lysed solid phase in an aqueous liquid phase, preferentially buffered,
e. liquid/solid separation and recovery of the liquid phase,
f. optional filtration of the liquid phase.
**characterized in that** the aqueous liquid phase of the resuspension step d) is a basic buffer selected from Tris buffer, arginine buffer or Tris-arginine buffer.

2. Bacterial extract according to claim 1, **characterized in that** the bacterium comprises at least one plasmid comprising sequence SEQ ID NO: 2, or any sequence having at least 80% identity with sequence SEQ ID NO: 2.

3. Bacterial extract according to one of claims 1 or 2, for use in the prevention and/or the treatment of cutaneous neurogenic inflammation.

4. Extract for use according to claim 3, **characterized in that** the cutaneous neurogenic inflammation includes sensitive skin and/or intolerant skin.

5. Cosmetic or dermatological composition comprising a bacterial extract according to one of claims 1 or 2 and at least one cosmetically or dermatologically acceptable excipient.

6. Cosmetic or dermatological composition according to claim 5, **characterized in that** it is suitable for topical application.

7. Dermatological composition according to claim 5 or 6, for use in the prevention and treatment of cutaneous neurogenic inflammation.

8. Composition for use according to claim 7, **characterized in that** the cutaneous neurogenic inflammation includes sensitive skin and/or intolerant skin.
